# EUROPEAN PATENT APPLICATION

(11) **EP 3 315 105 A1**
(43) Date of publication of application: **02.05.2018**
(21) Application number: 16814164.6
(22) Date of filing: 08.06.2016
(51) Int. Cl.: A61F 13/02, A45D 44/22

(54) **COSMETIC EYELID-SHAPING TAPE, METHOD FOR FORMING DOUBLE EYELID USING SAME, AND METHOD FOR PRODUCING SAID TAPE**

(30) Priority: 25.06.2015 JP 2015128049
(71) Applicant: artsbrains. co. Ltd., Shibuya-ku Tokyo 150-0001 (JP)
(72) Inventor: IWAGAKI Naoko, Tokyo 150-0001 (JP); KAMAGATA Masayuki, Tokyo 150-0001 (JP); TANIYAMA Jirou, Tokyo 150-0001 (JP)
(74) Representative: Carpmael, Robert Maurice Charles
(86) International application number: PCT/JP2016/067035
(87) International publication number: WO 2016/208393

(57) **Abstract**

[Object] In a piece of cosmetic eyelid-shaping tape used for performing pseudo shaping on an eyelid by forming a constricted portion on the eyelid by using elastic contractility when the tape-shaped member is stretched, to improve adhesiveness of the tape-shaped member to skin on an inner corner side of the eyelid of the eye (and/) or on an outer corner side of the eyelid of the eye.

[Solution] In a piece of cosmetic eyelid-shaping tape 1 that includes a tape-shaped member 4 including a base material piece 2 capable of being stretched in a longitudinal direction, the base material being formed of a long and narrow synthetic resin that has elastic contractility when stretched, and an adhesive layer 3 coated on both surfaces of the base material piece, and that forms a fold 91 in an upper eyelid by using elastic contractility of the base material piece, an entire adhesive area A in the tape-shaped member used for sticking the tape-shaped member on the eyelid is a width decreasing section in which a tape width of the tape-shaped member decreases from a first end 4a side towards a second end 4b side.

## Description

### Technical Field

The present invention relates to a piece of cosmetic eyelid-shaping tape for performing pseudo shaping on an eyelid, such as forming a fold of a pseudo double eyelid in an upper eyelid by forming a constricted portion in the eyelid by using elastically extendable and contractible characteristics of a tape-shaped member, specifically, using elastic contractility of a stretched base material piece formed of a stretchable synthetic resin constituting a tape-shaped member, a method for forming a double eyelid using the same, and a method for producing the cosmetic eyelid-shaping tape.

As cosmetics for forming a pseudo double eyelid on an upper eyelid without any surgery, there have been known a solution type (PTLs 1 to 3) of applying a solution on the skin of the upper eyelid, and a tape type (PTLs 4 to 8) of sticking a piece of adhesive tape on the skin of the upper eyelid.

Furthermore, the solution type and the tape type can be categorized mainly, in accordance with the method in which the double eyelid is formed, into a method (an adhesion method) in which a fold of the double eyelid is formed by bonding folded portions of skin in the upper eyelid with a piece of double-sided adhesive tape, an adhesive agent, or the like, and a method (a shutter method) in which a fold of the double eyelid is formed, for example, by applying and drying a solution on the skin of the upper eyelid or sticking an adhesive tape on the skin of the upper eyelid to form a stiff coating on the skin of the upper eyelid so that when the upper eyelid is opened, the skin of the upper eyelid is bent back at the upper rim of the coating.

However, the above-described double eyelid forming cosmetics of the related art forcibly form the fold of the double eyelid by using the bonding or coating with a solution or a piece of tape. Accordingly, there have been problems that the formed double eyelid tends to be unnatural, particularly, when the upper eyelid is closed, it is easily noticeable that the double eyelid forming cosmetics are used, the user easily feels discomfort, such as feeling of the skin being pulled, and other problems.

In order to resolve the above problems, a piece of double eyelid forming tape (PTL 9) that forms a double eyelid with a totally different method from the above-described methods of the related art has been proposed. The double eyelid forming tape is constituted by a tape-shaped member that is formed by coating an adhesive on a base material piece that can be elongated, and that has an elastically extendable and contractible property even after the elongation. In other words, the double eyelid forming tape uses the elastic contractility after the elongation of the base material piece described above that constitutes the tape-shaped member to form a recessed and groove-shaped constricted portion in the upper eyelid along the tape-shaped member and, as a result, when the upper eyelid is opened, the skin of the upper eyelid is bent back at the constricted portion in a natural manner and a fold of the double eyelid is formed. Furthermore, currently, the tape not only turns a single-edged eyelid into a double eyelid but also is used to perform various eye make-ups (in other words, pseudo shaping) on the eyelid, such as changing the width in the vertical direction, the position, and the shape of the fold of the double eyelid, and forming a so-called periorbital puffiness in the lower eyelid.

### Citation List

### Patent Literature

PTL 1: Japanese Unexamined Patent Application Publication No. 2007-106711
PTL 2: Japanese Registered Utility Model No. 3111511
PTL 3: Japanese Unexamined Patent Application Publication No. 02-188512
PTL 4: Japanese Registered Utility Model No. 3154139
PTL 5: Japanese Unexamined Patent Application Publication No. 10-304935
PTL 6: Japanese Unexamined Patent Application Publication No. 2005-334108
PTL 7: Japanese Unexamined Patent Application Publication No. 2007-111218
PTL 8: Japanese Unexamined Patent Application Publication No. 2009-195410
PTL 9: Japanese Patent No. 3277180

### Summary of Invention

### Technical Problem

In such a piece of cosmetic eyelid-shaping tape for performing pseudo shaping on the eyelid by forming a constricted portion in the eyelid using the elastic contractility when the base material piece constituting the tape-shaped member is stretched, the tape-shaped member is kept stretched and pulled while being abutted against and stuck on the eyelid when in use. However, the skin of the eyelid is formed with a complex curved surface curved along the eyeball, along the bones around the eyeball, and the like and, further, the skin of the eyelid has elasticity. Accordingly, it is not always easy to obtain a sufficient adhesiveness across the entire portion of the tape-shaped member described above in the longitudinal direction that is stuck on the eyelid with the sticking operation through abutting described above.

Because of the above, depending on the user, there are cases in which the portion in the tape-shaped member described above stuck on the eyelid easily comes off particularly at the end portion of the eye on the inner corner side and/or the end portion of the eye on the outer corner side.

Accordingly, the technical issue of the present invention is to provide a piece of cosmetic eyelid-shaping tape that is configured by a tape-shaped member including a base material piece formed of a stretchable synthetic resin and an adhesive layer coated on the base material piece, and that is provided for performing pseudo shaping on an eyelid by forming a constricted portion in the eyelid by using elastic contractility of the base material piece when the base material piece is stretched, in which the adhesiveness of the tape-shaped member is improved at the end portion of the eyelid on the inner corner side of the eye and/or the end portion of the eyelid on the outer corner side of the eye against the skin, a method for forming the double eyelid using the tape, and a method for producing the tape.

### Solution to Problem

In order to resolve the above issue, a piece of cosmetic eyelid-shaping tape of the present invention includes a tape-shaped member including a base material piece capable of being stretched in a longitudinal direction, the base material being formed of a long and narrow synthetic resin that has elastic contractility when stretched, and an adhesive layer coated on both surfaces or one surface of the base material piece, the cosmetic eyelid-shaping tape being used for performing pseudo shaping on an eyelid by using the elastic contractility of the base material piece to form a constricted portion in the eyelid, in which the tape-shaped member includes, on a first end side and a second end side of the tape-shaped member in the longitudinal direction, a pair of holding areas to be held with fingertips, and an adhesive area between the pair of holding areas, the adhesive area to be stuck on the eyelid by being stretched to a predetermined length, and in which the adhesive area includes only a single width decreasing section in which a width of the base material piece continuously decreases from the first end side towards the second end side, and the adhesive area does not have, with respect to the width decreasing portion, a portion on the first end side that is smaller in width than a largest width of the base material piece in the width decreasing section.

In such a piece of cosmetic eyelid-shaping tape according to the present invention, when the pair of holding areas of the tape-shaped member described above are held and the adhesive area is pulled in the longitudinal direction with the fingertips, the adhesive area becomes stretched as the base material piece becomes stretched, and the base material piece in the adhesive area becomes stretched sequentially from the portion where the width is smaller towards the portion where the width is larger. In the above, in the adhesive area described above, since the portion of the base material piece that has a larger width has a larger area of adhesion and a larger amount of adhesive agent, the adhesiveness to the skin is excellent, and since, simultaneously with the stretching of the adhesive area, the adhesive layers are stretched and become thinner, the portion that has a smaller elongation rate excels in adhesiveness to the skin.

In other words, the strength of the base material piece of the tape-shaped member described above against pulling becomes weaker as the cross-sectional area becomes smaller. Accordingly, the adhesive area described above is sequentially stretched from the second end side of the width decreasing section described above in which the width of the base material piece is smaller towards the first end side of the width decreasing section in which the width of the base material piece is larger. Moreover, the adhesive area does not include, on the first end side described above with respect to the width decreasing section described above, a portion in which the width is smaller than the largest width of the base material piece in the width decreasing section. Accordingly, when the adhesive area described above is stretched to the predetermined length described above, a more excellent adhesiveness can be obtained on the first end side of the adhesive area.

On the other hand, the width of the base material piece other than in the portion in the adhesive area described above where adhesiveness needs to be improved has been decreased further. Accordingly, when stretched and stuck on the eyelid, the tape-shaped member can be made unnoticeable as much as possible.

Accordingly, for example, in a case in which the entire area of the adhesive area described above in the longitudinal direction is formed with the width decreasing section described above, since a larger adhesive strength can be obtained on the first end side of the adhesive area, the adhesiveness to the eyelid at the end portion of the eyelid on the inner corner side of the eye or the end portion of the eyelid on the outer corner side of the eye can be improved by sticking the first end side of the adhesive area described above on either of the eyelid at the inner corner side of the eye and the eyelid at the outer corner side of the eye in which the tape-shaped member comes off more easily. Furthermore, for example, if a width increasing section in which the width of the base material piece continuously increases from the first end side towards the second end side is provided in the adhesive area described above on the second end side with respect to the width decreasing section in addition to the width decreasing section described above, adhesiveness to the eyelid can be improved both at the end portion of the eyelid on the inner corner side of the eye and at the end portion of the eyelid on the outer corner side of the eye.

Note that as described above, in a case in which the entire area of the adhesive area described above in the longitudinal direction is formed with the width decreasing section described above, it is desirable that the tape-shaped member described above is formed in a triangle or a trapezoid in plan view in which the width decreases from the first end towards the second end, because the yield can be improved and the manufacturing cost can be suppressed.

In the cosmetic eyelid-shaping tape described above, it is preferable that the base material piece includes at least a first base material layer, and that the first base material layer of the base material piece is elongated when the adhesive area is stretched to the predetermined length, and has elastic contractility in an elongated state as well.

In the above, it is preferable that the base material piece described above is constituted by a laminated body of the first base material layer described above formed of a polyolefin resin, and a second base material layer formed of an elastomer resin. In such a case, it is more preferable that the first base material layer described above is formed of a low-density polyethylene resin, and the second base material layer formed of a polyurethane resin.

By so doing, the elastic contractility of the first base material layer and the elastic contractility of the second base material layer can be combined, and the elastic contractility of the base material piece, in other words, the elastic contractility of the tape-shaped member, can be made to widely respond to various states of the eyelid from a state of the eyelid in which the constricted portion is easily formed to a state of the eyelid in which formation of the constricted portion is difficult.

Furthermore, in the present invention, the cosmetic eyelid-shaping tape may be further formed of a synthetic resin that has easy release characteristics against the adhesive layer, and may include a release sheet piece that covers the adhesive layer and that is stuck on the holding areas and the adhesive area of the tape-shaped member, and the release sheet piece may include an easily breakable portion at a portion corresponding to the adhesive area, the easily breakable portion being cut into a depth reaching a middle in a thickness direction while being provided so as to extend in the width direction, the easily breakable portion being broken by pulling in the longitudinal direction.

In such a case, by holding the holding areas of the tape-shaped member described above from above the release sheet piece described above with the fingertips, and by pulling the adhesive area in the longitudinal direction, the easily breakable portion of the release sheet piece becomes broken and the adhesive area is stretched and, as a result, the adhesive area portion is exposed and becomes visible between the release sheet pieces that have been separated at the easily breakable portion described above.

Furthermore, the cosmetic eyelid-shaping tape described above may be further formed of, in place of the release sheet described above, a film having easy release characteristics against the adhesive layer, and may include a release film piece that covers the adhesive layer and that is stuck on the adhesive area of the tape-shaped member, the pair of holding areas may be nonadhesive, the release film piece may be formed shorter than the tape-shaped member, the holding areas may be exposed on the first end side and the second end side, and the release film piece may extend on the tape-shaped member.

In such a case, by holding with the fingertips the nonadhesive holding areas where the tape-shaped member has been exposed, and by pulling the adhesive area in the longitudinal direction, simultaneously with the above-described adhesive area of the tape-shaped member being stretched, the release film piece described above is separated from the adhesive area and, as a result, the adhesive area is exposed and becomes visible.

A method for forming a double eyelid using the cosmetic eyelid-shaping tape described above according to the present invention includes the following steps:
1. a step of stretching an adhesive area of the tape-shaped member to the predetermined length by holding and pulling a pair of holding areas of the tape-shaped member in the longitudinal direction;
2. a step of sticking a portion or all of the adhesive area stretched to the predetermined length in step 1 on an upper eyelid with the adhesive by abutment against the upper eyelid while the tape-shaped member is kept in a pulled state;
3. a step of forming a constricted portion along the adhesive area in the upper eyelid by releasing the pulling of the tape-shaped member and elastically shrinking a base material piece in the adhesive area that has been stuck on the upper eyelid; and
4. a step of forming a fold of the double eyelid by skin of the upper eyelid being bent back at the constricted portion when the upper eyelid is opened.

In other words, in a state in which the tape-shaped member described above is kept pulled, by abutting the predetermined length described above, in other words, by abutting a portion or all of the adhesive area described above that is stretched to a length that is needed to abut against and stuck on the upper eyelid is abutted against and stuck on the skin of the upper eyelid that has elasticity, and by releasing the pulling, the portion of the base material piece in the adhesive area that has actually been stuck on the eyelid countering the restoring force of the skin of the eyelid elastically shrinks in the stuck portion of the adhesive area and as if bit into the skin of the upper eyelid to form a constricted portion along the stuck portion. As a result, when the upper eyelid is opened, the skin of the upper eyelid is bent back downwards at the constricted portion described above and a fold of the double eyelid is formed.

Moreover, the cosmetic eyelid-shaping tape described above according to the present invention can be fabricated with the producing method including the following steps:
1. a step of preparing an adhesive sheet on which an adhesive is coated on both surfaces or one surface of a base material sheet formed of a synthetic resin that forms the base material piece; and
2. a step of obtaining the cosmetic eyelid-shaping tape including the tape-shaped member by cutting the adhesive sheet with a cutting blade.
In the above, as described above, if the tape-shaped member is, in particular, formed in a triangle or a trapezoid, not only the manufacturing is facilitated but also the yield improves.

Note that in the present invention, the "predetermined length" refers to a length that can be abutted against and stuck on the eyelid while the adhesive area is pulled and kept stretched. Furthermore, "elongate" refers to plastically deforming the synthetic resin piece such as, for example, the first base material layer that constitutes the base material piece, by stretching through pulling when the cosmetic eyelid-shaping tape described above is in use. Furthermore, "elastic contractility" refers to elastic characteristics of the synthetic resin piece of the base material piece and the like described above that shrink with elastic force immediately after the above pulling has been released, and contributes to the formation of the constricted portion needed to perform the pseudo shaping on the eyelid.

### Advantageous Effects of Invention

As described above, the present invention is capable of providing a piece of cosmetic eyelid-shaping tape for performing pseudo shaping on an eyelid by forming a constricted portion in the eyelid by using elastic contractility of the tape-shaped member (in other words, the elastic contractility of the base material piece formed of a synthetic resin constituting the tape-shaped member) when the tape-shaped member is stretched to a predetermined length, in which an improvement in the adhesiveness of the tape-shaped member at the end portion of the eyelid on the inner corner side of the eye and/or the end portion of the eyelid on the outer corner side of the eye against the skin, a method for forming the double eyelid using the tape, and a method for producing the tape. Brief Description of Drawings

[Fig. 1] Fig. 1 is a schematic front view illustrating a first embodiment of a piece of cosmetic eyelid-shaping tape according to the present invention.
[Fig. 2] Fig. 2 is an enlarged D-D cross-sectional view of the tape illustrated in Fig. 1.
[Fig. 3] Fig. 3 is a plan view of the tape illustrated in Fig. 1.
[Fig. 4] Fig. 4 is an exploded perspective view of the tape illustrated in Fig. 1.
[Fig. 5] Fig. 5 is an enlarged view of an adhesive area A of the tape illustrated in Fig. 1.
[Fig. 6] Fig. 6 is a schematic view 1 illustrating a method of use of the cosmetic eyelid-shaping tape according to the present invention.
[Fig. 7] Fig. 7 is a schematic view 2 illustrating a method of use of the cosmetic eyelid-shaping tape according to the present invention.
[Fig. 8] Fig. 8 is a schematic view 3 illustrating a method of use of the cosmetic eyelid-shaping tape according to the present invention.
[Fig. 9] Fig. 9 is a schematic view 4 illustrating a method of use of the cosmetic eyelid-shaping tape according to the present invention.
[Fig. 10] Fig. 10 is a schematic view 5 illustrating a method of use of the cosmetic eyelid-shaping tape according to the present invention.
[Fig. 11] Fig. 11 is a schematic view 6 illustrating a method of use of the cosmetic eyelid-shaping tape according to the present invention.
[Fig. 12] Fig. 12 is a schematic view 7 illustrating a method of use of the cosmetic eyelid-shaping tape according to the present invention.
[Fig. 13] Fig. 13 is a schematic view 1 illustrating a method for producing the cosmetic eyelid-shaping tape according to the present invention.
[Fig. 14] Fig. 14 is a schematic view 2 illustrating a method for producing the cosmetic eyelid-shaping tape according to the present invention.
[Fig. 15] Fig. 15 is a schematic view 3 illustrating a method for producing the cosmetic eyelid-shaping tape according to the present invention.
[Fig. 16] Fig. 16 illustrates plan views (a) to (h) indicating modifications of the first embodiment described above.
[Fig. 17] Fig. 17 is a schematic front view illustrating a second embodiment of a piece of cosmetic eyelid-shaping tape according to the present invention.

### Description of Embodiments

Hereinafter, embodiments of a piece of cosmetic eyelid-shaping tape according to the present invention will be described in detail with reference to the drawings. Note that herein, while a piece of cosmetic eyelid-shaping tape for forming a single-edged eyelid into a double eyelid is specifically described by citing a piece of double eyelid forming tape as an example, not limited to the above, the cosmetic eyelid-shaping tape according to the present invention can be applied to various pieces of tape for performing pseudo shaping on an eyelid by forming a constricted portion in the eyelid using elastic contractility of a stretched tape-shaped member to change a position and a shape or an up-down direction width of a fold of the double eyelid, to form a so-called periorbital puffiness in the lower eyelid, and the like.

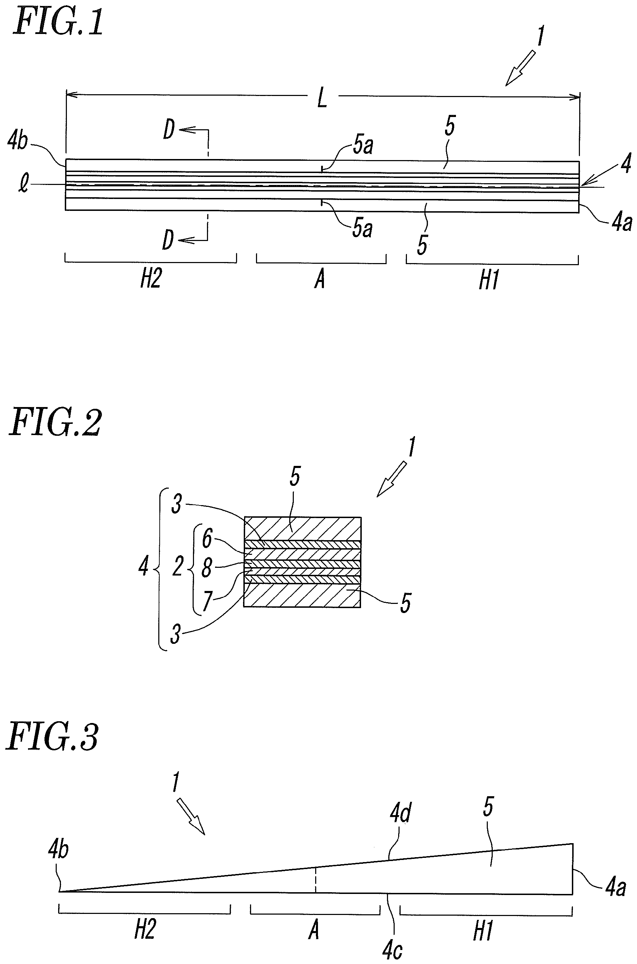

As illustrated in Figs. 1 to 4, a piece of double eyelid forming tape 1 according to the present invention includes a tape-shaped member 4 including a base material piece 2 that is capable of being stretched in the longitudinal axis 1 direction and that is formed of a long and narrow synthetic resin that has elastic contractility when stretched to a use length, in other words, to a predetermined length suitable for a sticking operation on the upper eyelid, and adhesive layers 3 and 3 coated on both surfaces of the base material piece 2, and forms a fold of the double eyelid using the elastic contractility during the above-described stretching of the base material piece 2.

The base material piece 2 described above is formed in a long and narrow tape shape with a laminated body of a first base material layer 6 and a second base material layer 7 formed of a synthetic resin, and is configured so that even if, after being pulled and stretched to the predetermined length described above, the tensile force is released, the stretched length does not return to its original length before pulling. In the above, the base material layers 6 and 7 are laminated to each other with an adhesion layer 8. Furthermore, the adhesive layers 3 and 3 described above are formed by coating, on the entire surfaces, an adhesive on both surfaces on the front and back of the base material piece 2 described above. Note that the adhesive layer 3 does not necessary have to be formed on both surfaces of the base material piece 2 and may be formed on only one surface.

The tape-shaped member 4 that is constituted by the base material piece 2 and the adhesive layers 3 and 3 described above includes a first end 4a and a second end 4b at both ends in the longitudinal axis 1 direction thereof. Furthermore, as well as a pair of holding areas H1 and H2 on the first end 4a side and the second end 4b side described above used for holding with the fingertips, the tape-shaped member 4 includes, between the pair of holding areas H1 and H2, an adhesive area that is stretched to the predetermined length described above and that is used for sticking on the eyelid.

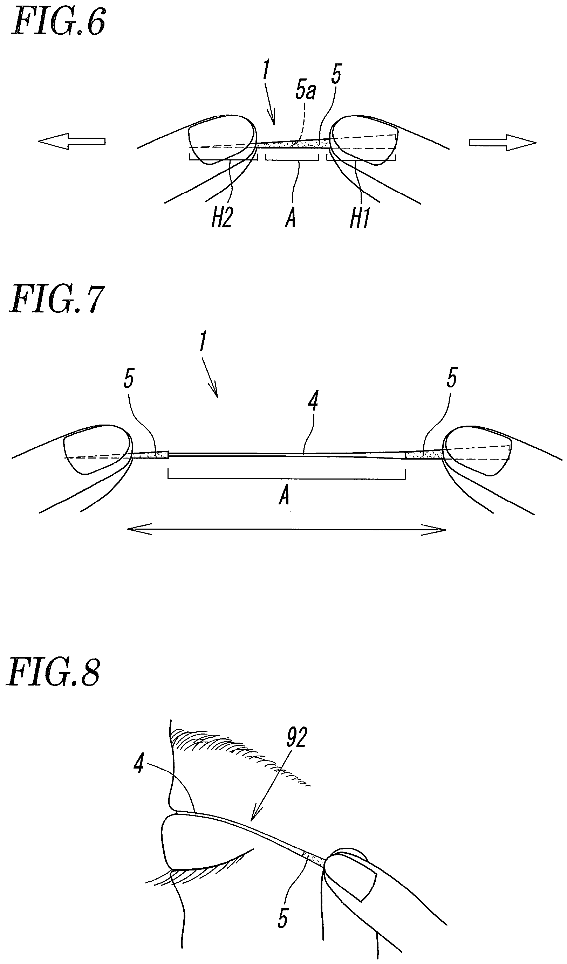

Furthermore, in forming a fold of the double eyelid in the upper eyelid using the double eyelid forming tape constituted by the tape-shaped member 4 described above, the pair of holding areas H1 and H2 described above of the tape-shaped member 4 are held and pulled in the longitudinal axis 1 direction with the fingertips so that the adhesive area A described above is stretched to the predetermined length described above that is longer than the lateral length of the eyelid that is at least from the outer corner of the eye to the inner corner of the eye (see Fig. 6 and Fig. 7). Subsequently, in a state in which the adhesive area A described above is kept stretched at the predetermined length described above (kept in a tensed state by the tension), a portion of or the entire adhesive area A is abut against and stuck on a virtual line that is to form a fold 91 of the pseudo double eyelid (in other words, to form a constricted portion 90) in the upper eyelid (see Fig. 8).

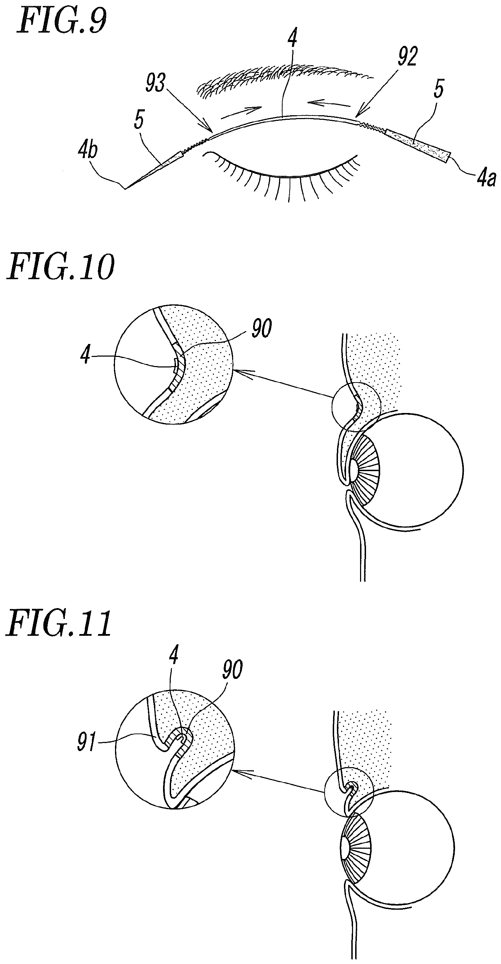

Subsequently, when the fingers are released from the holding areas H1 and H2 described above and the pull of the tape-shaped member 4 is released, a portion (hereinafter, referred to as a "stuck portion") in the adhesive area A described above that has actually been stuck on the virtual line of the eyelid described above countering the restoring force of the skin of the upper eyelid, which is elastic, elastically shrinks (see Fig. 9) so as to bite into the skin of the upper eyelid, and the constricted portion 90 is formed along the virtual line described above (in other words, along the stuck portion described above) (see Fig. 10). In the above, the stuck portion of the tape-shaped member 4 is stuck on the bottom portion of the constricted portion 90.

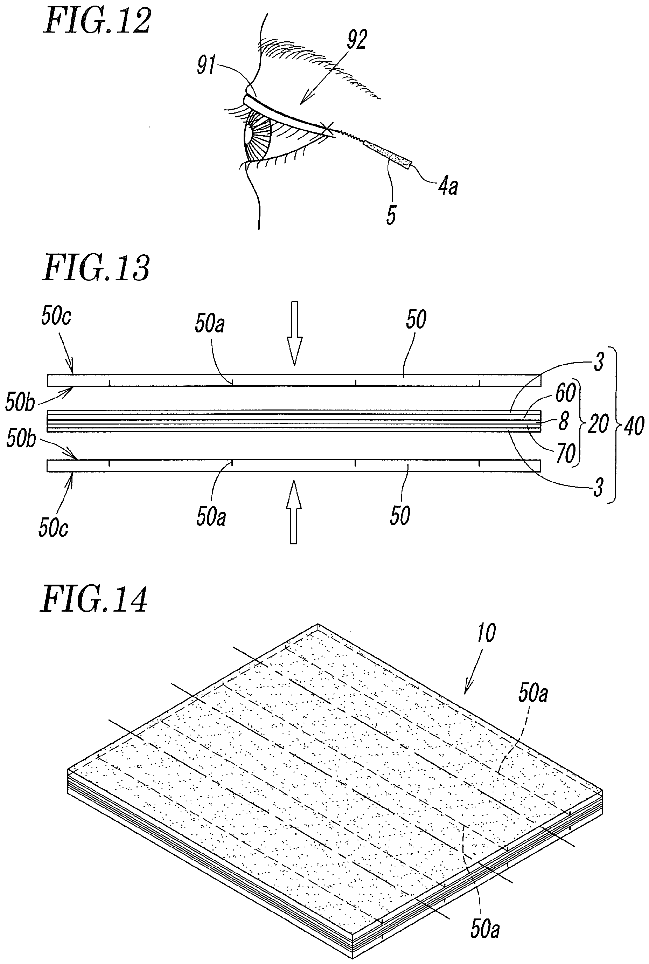

As a result, when the upper eyelid is opened, the skin of the upper eyelid is folded at the constricted portion 90 (in other words, the skin of the upper eyelid above the constricted portion 90 is bent back downwards at the constricted portion 90), and the fold 91 of the double eyelid is formed (see Fig. 11 and Fig. 12). In other words, it can be said that in a state in which the tape-shaped member 4 described above (specifically, the base material piece 2 of the tape-shaped member 4) is pulled and stretched to the predetermined length described above, there is elastic contractility that contributes to the formation of the constricted portion 90 required to form the fold of the double eyelid.

Note that after sticking the adhesive area A of the tape-shaped member 4 to the upper eyelid, excess portions such as the holding areas H1 and H2 that have become unneeded are cut and removed with a cutter, a pair of scissors, or the like (see x mark in Fig. 12). In so doing, both end portions of the tape-shaped member 4, which have been stuck on the upper eyelid and which have remained, are an end portion of the eye on an outer corner side 92 and an end portion of the eye on an inner corner side 93 of the present application and, furthermore, after the above, the shape of the double eyelid may be adjusted or the stuck tape-shaped member 4 may be made to fit onto the skin with a pusher (not shown) or the like.

Incidentally, as described above, in the double eyelid forming tape that forms the fold of the double eyelid using the elastic contractility while the base material piece included in the tape-shaped member is stretched, the tape-shaped member, while in use, kept in a stretched and pulled state is abutted against the virtual line described above of the upper eyelid and is stuck. However, the skin of the upper eyelid is formed with a convex surface curved along the eyeball and has elasticity and, furthermore, the bone around the eyeball affects the abutting and sticking operation described above; accordingly, it is not always easy to abut the tape-shaped member described above against the entire virtual line described above with uniform pressing force and obtain sufficient adhesiveness across the entire the tape-shaped member in the longitudinal direction in the portion stuck on the upper eyelid.

Because of the above, depending on the user, there are cases in which the portion in the tape-shaped member described above stuck on the upper eyelid, particularly the end portion of the eye on the outer corner side and/or the end portion of the eye on the inner corner side, easily comes off.

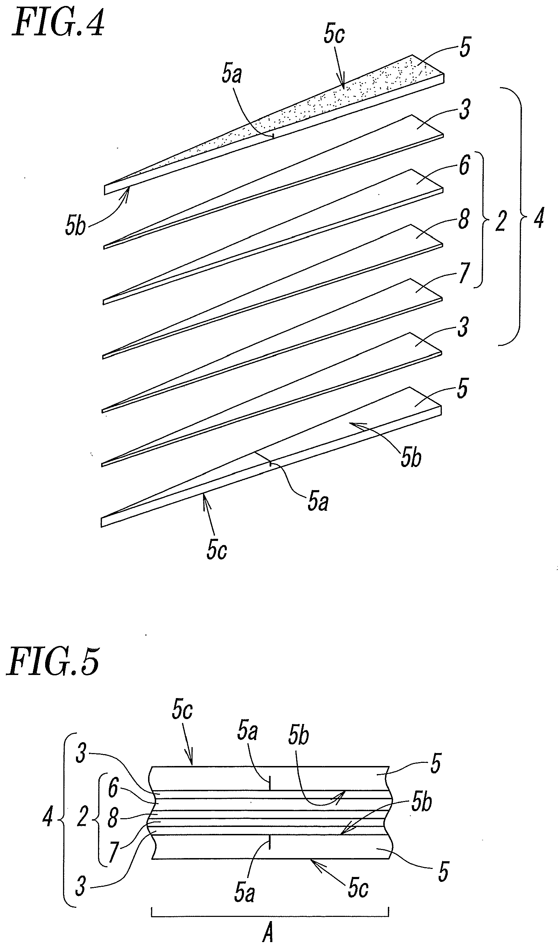

Accordingly, in the above-described double eyelid forming tape 1, as illustrated in Fig. 3 and Fig. 4, the planar shape of the tape-shaped member 4, in other words, the planar shape of the base material piece 2 is triangular in which the width continuously decreases across the entire length from the first end 4a described above towards the second end 4b described above. Specifically, it is a right angled triangle formed so that a first lateral edge surface 4c and a second lateral edge surface 4d that extend in the longitudinal direction of the tape-shaped member 4 form an acute angle at the second end 4b described above, and the first lateral edge surface 4c and the edge surface of the first end 4a described above form a right angle. By so doing, the entire area of the adhesive area A described above in the longitudinal direction becomes a width decreasing section in which the width continuously decreases from the above-described first end 4a side towards the second end 4b side.

Note that as it will be described later, the entire area of the adhesive area A does not have to be a width decreasing section, and it is only sufficient that the adhesive area A has only one section of the above width decreasing section, and does not have, on the above-described first end 4a side with respect to the width decreasing section, a portion in which the width is smaller than the largest width of the base material piece 2 in the width decreasing section (in other words, the largest width of the tape-shaped member 4 in the width decreasing section).

In the above-described double eyelid forming tape 1, when the pair of holding areas H1 and H2 of the tape-shaped member 4 described above are held and the adhesive area A is pulled in the longitudinal axis 1 direction with the fingertips, as described above, the adhesive area A becomes stretched as the base material piece 2 becomes stretched, and the base material piece 2 in the adhesive area A becomes stretched sequentially from the portion where the width is smaller towards the larger portion. In the above, in the adhesive area A described above, since the portion of the base material piece 2 described above that has a larger width has a larger area of adhesion and a larger amount of adhesive agent, the adhesiveness to the skin is excellent, and since, simultaneously with the stretching of the adhesive area A, the adhesive layers 3 are stretched and become thinner, the portion that has a smaller elongation rate excels in adhesiveness to the skin.

In other words, in the base material piece 2 of the tape-shaped member 4 described above, since the portion where the cross-sectional area is smaller has a weaker strength against pulling, the adhesive area A of the tape-shaped member 4 formed in a triangle as described above becomes stretched sequentially from the above-described second end 4b side in which the width of the base material piece 2 is smaller towards the first end 4a side in which the width of the base material piece 2 is larger. As a result, when the adhesive area A is stretched to the predetermined length described above, a more excellent adhesiveness can be obtained on the first end 4a side of the adhesive area A.

Accordingly, by sticking the first end 4a side of the adhesive area A described above on either the outer corner side 92 of the eye and the inner corner side 93 of the eye of the upper eyelid in which the tape-shaped member comes off more easily, the adhesiveness of the stuck portion to the upper eyelid can be improved in the end portion of the eye on the outer corner side 92 or the end portion of the eye on the inner corner side 93. On the other hand, since the width of the base material piece 2 becomes smaller on the above-described second end 4b side of the adhesive area A described above, when stretched and stuck on the upper eyelid, the tape-shaped member 4 can be made to be more unnoticeable. In the example of use in Fig. 8 and Fig. 9, the first end 4a side of the adhesive area A described above is arranged on the outer corner side 92 of the eye, and the second end 4b side of the adhesive area A described above is arranged on the inner corner side 93 of the eye.

Furthermore, when the tape-shaped member 4 is triangular, manufacturing thereof is easier and the yield can be improved; accordingly, the manufacturing cost can be suppressed.

Note that the first base material layer 6 of the base material piece 2 described above is formed of a synthetic resin film piece excluding elastomer resin or, preferably, a polyolefin resin film piece that, when stretched to the predetermined length described above by pulling the adhesive area A described above, is elongated, and has elastic contractility after being elongated as well. Moreover, in such a polyolefin resin film piece, a low-density polyethylene film piece is particularly preferable from the viewpoint of stretchability, shrinkage characteristics after the elongation, and the like. Furthermore, the polyolefin resin film piece described above, preferably, is a polyolefin resin film piece that has been axially stretched in the longitudinal axis 1 direction during manufacturing of the film in an in a nonoriented manner or at an extremely low draw ratio, and is a polyolefin resin film piece that, when held and stretched with the fingertips, elongates with a necking.

As described above, by forming the first base material layer 6 with a polyolefin resin film that is nonoriented or with an extremely low draw ratio in the longitudinal axis 1 direction, in the course of stretching the adhesive area A to the predetermined length described above, the elongations of the first base material layer 6 is suppressed on the second end 4b side of the adhesive area A where the width of the tape-shaped member 4 is narrow. Accordingly, the width of the base material piece 2, in other words, the width of the tape-shaped member 4, does not become excessively small and the required adhesiveness can be obtained.

Furthermore, it is preferable that the second base material layer 7 of the base material piece 2 described above is formed of an elastomer resin film piece that has a shrinkage force that is smaller than that of the first base material layer 6 (preferably, 1/2 or smaller) or a larger shrinkage factor when the adhesive area A described above is stretched to the predetermined length that is the same as that of the first base material layer 6 described above by pulling; however, among the above, it is more preferable to be formed of a polyurethane resin film piece from the viewpoint of combination with the low-density polyethylene film piece.

As described above, by combining the elastic contractility of the first base material layer 6 and the elastic contractility of the second base material layer 7, the elastic contractility of the base material piece 2, in other words, the elastic contractility of the tape-shaped member 4, can, while limiting the gross-sectional area of the base material piece 2, be made to widely respond to various states of the upper eyelid from an eyelid that is easily doubled to an eyelid that is difficult to be doubled. As in the present embodiment, in a case in which the first base material layer 6 described above is formed of low-density polyethylene described above, and the second base material layer 7 described above is formed of polyurethane resin, a preferable elastic characteristics of the base material piece 2 can be obtained by setting the thickness of the first base material layer 6 described above in the range of about 70 to 80 µm, and the thickness of the second base material layer in the range of about 7 to 9 µm, for example.

Since the double eyelid forming tape in which the base material piece 2 is formed of such a laminated body is disclosed in a previous application of the applicant (Japanese Unexamined Patent Application Publication No. 2014-212847), it is incorporated by reference into the present application and, herein, further specific description thereof is omitted. Furthermore, while the combination of the base material layers forming the base material piece 2 does not necessary have to be the one described above, it is desirable that at least the first base material layer 6 described above be included.

Note that the first base material layer 6 and the second base material layer 7 described above do not have to, as described above, be laminated with the adhesion layer 8 interposed therebetween and, the two base material layers 6 and 7 can be integrally formed by directly binding the first base material layer 6 and the second base material layer 7 to each other such as, for example, coating and solidifying the second base material layer 7 on the first base material layer 6, or integrating the base material layers 6 and 7 by hot pressing.

Moreover, the base material piece 2 does not necessarily have to be formed of a laminated body of synthetic resin film pieces and maybe formed of a single layer of a synthetic resin film piece. In such a case, the base material piece 2 is, preferably, formed of the first base material layer 6 and, more preferably, is formed of a low-density polyethylene film piece in particular.

The adhesive forming the adhesive layers 3 and the adhesion layer 8 described above is not particularly limited as long as it is suitable for the human skin; however, the same acrylic adhesive is preferably used. Furthermore, each of the adhesive layers 3 and 3 and the adhesion layer 8 may be formed of adhesives of different types and thicknesses. Moreover, while the adhesive layer 3 described above is provided across the entire surface of each of the two surfaces or one of the surfaces of the base material piece 2, for example, it may be formed only on the portion of the base material piece 2 corresponding to the adhesive area A of the tape-shaped member 4 described above.

Moreover, regarding the sizes of the tape-shaped member 4 described above, the length of the adhesive area A described above is, since it relies on the preference of the user, not limited in particular; however, considering the actual operability and the like, preferably, the length before being stretched is in the range of about 0.5 to 2.0 cm, and the length after being stretched is in the range of about 4.0 to 16.0 cm. Standardly, the length before being stretched is about 1.0 cm and the length after being stretched is about 8.0 cm. Together with the operability, when considering a more effective elastic contractility of the synthetic resin piece in the above-described base material piece 2 and the like, the stretching factor is preferably about 8.0 times; however, it is not limited to the above in particular and it goes without saying that it may be changed in accordance with the preference of the user.

Considering operability, the pair of holding areas H1 and H2 described above is preferably in the range of about 0.5 to 1.0 cm.

Accordingly, the length L of the tape-shaped member 4 described above is preferably in the range of about 1.5 to 4.0 cm and, more preferably, is about 2.5 to 3.0 cm.

Moreover, in the adhesive area A described above, the width of the tape-shaped member 4 (in other words, the width of the base material piece 2) is preferably, for practical purposes, 0.6 mm or more at the narrowest portion and 5.0 mm or less at the widest portion, and in a case of a right angled triangle as illustrated in Fig. 3, the angle formed between the first lateral edge surface 4c and the second lateral edge surface 4d described above is preferably in the range of about 4 to 26 degrees when considering each of the sizes described above.

A first embodiment of the double eyelid forming tape 1 constituted by the tape-shaped member 4 in the above described manner will be described next with reference to Fig. 1 to Fig. 5.

The double eyelid forming tape according to the first embodiment of the present invention includes the rectangular tape-shaped member 4 described above, and two release sheet pieces 5 and 5 stuck on the surfaces of the adhesive layers 3 and 3 described above that face the external sides that are on the opposite sides with respect to the base material piece 2. The release sheet pieces 5 are formed of a synthetic resin that has easy release characteristics against the adhesive layers 3 described above, have the same shape as that of the tape-shaped member 4 described above, and are stuck on so as to cover the entire surface of the adhesive layers 3 described above.

Moreover, the release sheet pieces 5 include, at the middle in the longitudinal axis 1 direction thereof (in other words, at positions corresponding to the adhesive area A in the longitudinal axis 1 direction), easily breakable portions 5a that are broken by the pulling operation in the longitudinal axis 1 direction. Each easily breakable portion 5a is provided from a first surface 5b (the inner surface in the drawing), which is in contact with the adhesive layer 3 of the release sheet piece 5, towards the second surface 5c (the outer surface in the drawing) side, cuts (in other words, slit) into the middle of the thickness of the release sheet piece 5, and is provided in the width direction across the entire width.

Note that the form of each easily breakable portion 5a is not limited to a cut and, for example, may be formed in a form that locally reduces the thickness of the release sheet piece 5, such as a V-shaped notch.

Note that the release sheet pieces 5 described above are integrally molded by a silicone resin that has both the easy release characteristics against the adhesive layers 3 and 3, and the brake characteristics in the easily breakable portions 5a described above. The larger the hardness of the silicone resin the more preferable since it excels in the break characteristics described above, and the thickness thereof is preferably about 0.3 to 0.5 mm.

Furthermore, the first surface 5b of the release sheet piece 5 described above that is in contact with the adhesive layers 3 is formed as a mirror surface, and the second surface 5c on the opposite side is formed as a micro rough surface, and in the manufacturing stage, by turning over the first surface 5b and the second surface 5c of the release sheet piece 5, the surface in contact with the adhesive 3 is switched; accordingly, the release characteristics (adhesiveness) between the release sheet piece 5 and the adhesive 3 can be adjusted.

In using such a piece of double eyelid forming tape 1 according to the first embodiment, the pair of holding areas H1 and H2 of the tape-shaped member 4 described above are held with the release sheet pieces 5 and 5 interposed in between (see Fig. 6) and the tape-shaped member 4 is pulled in the longitudinal axis 1 direction. By so doing, the release sheet pieces 5 and 5 are broken at the easily breakable portions 5a and 5a described above and are separated from the adhesive layers 3 and 3 in the adhesive area A of the tape-shaped member 4 and, as a result, the adhesive area A of the tape-shaped member 4 is stretched and the entirety thereof can be exposed (see Fig. 7). The subsequent method for use and the functions and effects are as described above; accordingly, in order to avoid redundant description, description thereof is omitted herein.

A method for producing the double eyelid forming tape according to the first embodiment of the present invention will be described next with reference to Fig. 13 to Fig. 15.

First, as illustrated in Fig. 13, an adhesive is coated on the entire front and back surfaces of a base material film 20 formed of a laminated body of a first layer film (a low-density polyethylene film, for example) 60 for forming the first base material layer 6 described above and a second layer film (a polyurethane resin film, for example) 70 for forming the second base material layer 7 described above so as to prepare a double-sided adhesive sheet 40 in which the adhesive layers 3 and 3 described above are formed.

The double-sided adhesive sheet 40 can be prepared, for example, by superposing a double-sided adhesive film formed by forming the adhesive layer 3 and the adhesion layer 8 described above by coating an adhesive on the entire two surfaces of the first layer film 60 described above and a single-sided adhesive film formed by forming the adhesive layer 3 descried above by coating an adhesive on the entire single surface of the second layer film 70 described above, so that the adhesive layers 3 of the films are on the outside and by adhering each other with the adhesion layer 8.

Furthermore, meanwhile, a release sheet piece 50 in which a first surface 50b (the inner surface in the drawing) is a mirror surface and a second surface 50c (the outer surface in the drawing) on the opposite side is a rough surface is prepared by thermoforming a silicone resin with high hardness, and easily breakable lines 50a that are parallel to each other at equal intervals are formed in the first surface 50b described above by cutting to the middle in the thickness direction from the first surface 50b towards the second surface 50c.

Note that the double-sided adhesive sheet 40 and the release sheet 50 described above are preferably formed so as to have the same longitudinal and lateral size.

Furthermore, a laminated sheet 10 illustrated in Fig. 14 is prepared by, as illustrated in Fig. 13, opposing the first surface 50b of each release sheet 50 to the corresponding adhesive layer 3 of the double-sided adhesive sheet 40 and by sticking the two release sheets 50 and 50 and the double-sided adhesive sheet 40 to each other. In the above, the easily breakable lines 50a and 50a of the release sheets 50 and 50 are parallel to each other and are disposed so as to oppose each other.

Note that in a case in which the first layer film 60 of the double-sided adhesive sheet 40 described above is a polyolefin resin film, preferably, the film is nonoriented or is with an extremely low draw ratio in the width direction that forms a right angle with the feeding direction during manufacturing, and the release sheet 50 and 50 described above and the double-sided adhesive sheet 40 are stuck together so that the width direction of the film forms a right angle between the easily breakable lines 50a of the release sheets 50 described above.

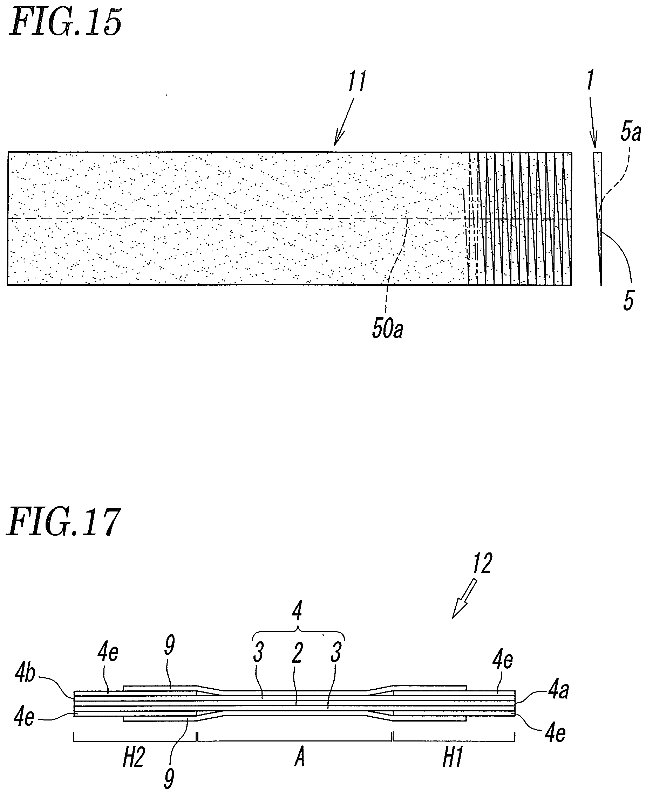

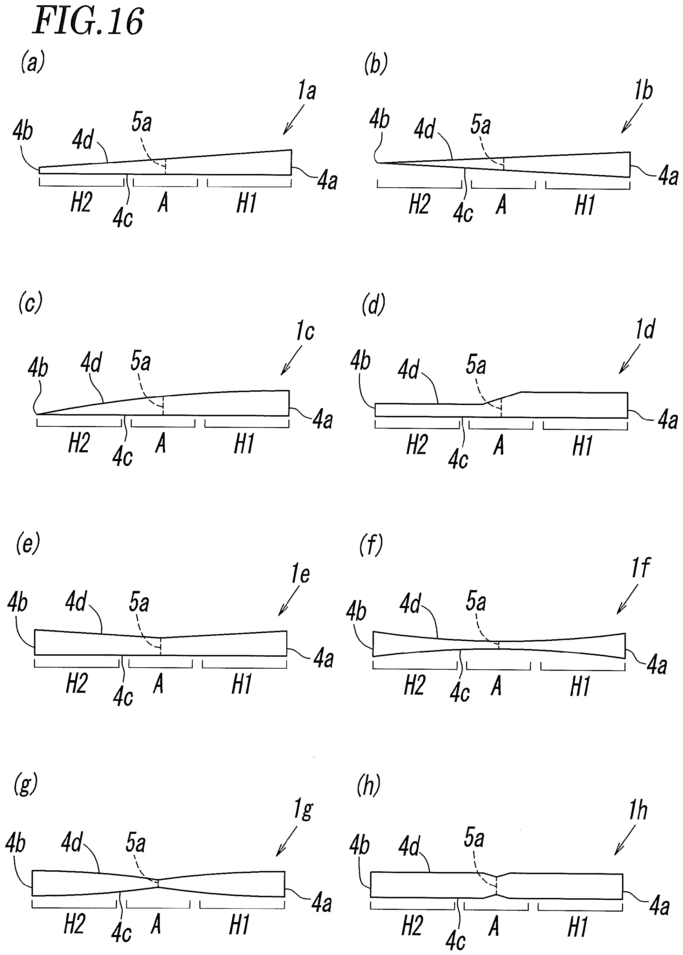

Subsequently, as illustrated in Fig. 15, the laminated sheet 10 described above is, at the middle between the adjacent easily breakable lines 50a, cut parallel to the easily breakable lines 50a to form long and narrow strip-shaped laminated sheets 11 and, furthermore, as illustrated in Fig. 16, the strip-shaped laminated sheets 11 are cut further thin by a combination between a cutting blade (not shown) that extends in a direction perpendicular to the easily breakable lines 50a described above and a cutting blade (not shown) that extends so as to form an acute angle with the cutting blade. As a result, the easily breakable portions 5a described above are provided at the middle in the longitudinal direction and pieces of double eyelid forming tape 1 described above that each form a right angled triangle can be obtained.

Referring to Fig. 16, modifications of the double eyelid forming tape according to the first embodiment of the present invention will be described next. Note that the double eyelid forming tape 1 according to the first embodiment described above and the modifications are different from each other mainly in the planar shapes (particularly, the shape of the adhesive area A), and the other configurations, and the functions and effects are practically the same.

Accordingly, herein, in order to avoid redundancy, description of the configurations and the functions and effects that are practically the same as those of the double eyelid forming tape 1 described above is omitted.

First, in pieces of double eyelid forming tape 1a to 1c illustrated in (a) to (c) of Fig. 16, similar to the double eyelid forming tape 1 according to the first embodiment described above, the width of the tape-shaped member 4 continuously decreases across the entire length from the first end 4a towards the second end 4b. Accordingly, in the longitudinal direction, the entire area of the adhesive area A becomes a width decreasing section in which the width continuously decreases from the above-described first end 4a side towards the second end 4b side. The tape 1a of (a) formed in a trapezoid each the first lateral edge surface 4c of the tape-shaped member 4 forms a right angle with the edge surface of the first end 4a. Furthermore, the tape 1b of (b) is formed in an isosceles triangle in which the lengths of the first and second lateral edge surfaces 4c and 4d are the same. Furthermore, the tape 1c of (c) is formed such that the first lateral edge surface 4c forms a right angle with the edge surface of first end 4a, and the second lateral edge surface 4d is a smooth curved surface protruding outwardly.

In a piece of tape 1d of (d) of Fig. 16, the adhesive area A of the tape-shaped member 4 includes, in the end portions thereof on the first end 4a side and the second end 4b side, width uniform sections that are each formed with a width that is the same as a corresponding one of the adjacent holding areas H1 and H2, and only one section of the width decreasing section that is interposed between the width uniform sections and in which the width continuously decreases from the first end 4a side towards the second end 4b side. Note that the easily breakable portion 5a of the release sheet piece 5 described above is disposed at the middle of the width decreasing section described above. Furthermore, the width of the holding area H1 is larger than the width of the holding area H2.

The above pieces of double eyelid forming tape 1a to 1d are, similar to the tape 1 described above, capable of providing a superior adhesiveness to the eyelid on the first end 4a side of the adhesive area A when extended in the longitudinal direction, and when extended and stuck on the eyelid, can improve the adhesiveness to the eyelid in either of the end portion of the eye on the outer corner side 92 and the end portion of the eye on the inner corner side 93.

In pieces of tape 1e to 1h of (e) to (h) of Figs. 16, the width of the adhesive area A of the tape-shaped member 4 continuously decreases from the first end 4a side and the second end 4b side thereof towards the middle in the longitudinal direction, and is the smallest at the position of the easily breakable portion 5a at the middle in the longitudinal direction. Accordingly, in the longitudinal direction, the adhesive area A forms a width decreasing section in the portion on the first end 4a side with respect to the easily breakable portion 5a that continuously decreases from the first end 4a side towards the second end 4b side. Meanwhile, the portion on the second end 4b side with respect to the easily breakable portion 5a forms a width increasing section that continuously increases from the first end 4a side towards the second end 4b side.

In the tape 1e in (e), the first lateral edge surface 4c of the tape-shaped member 4 forms right angles with the edge surfaces of the first end 4a and the second end 4b, and the second lateral edge surface 4d inclines in a straight manner in plan view from the first end 4a and the second end 4b towards the easily breakable portions 5a at the middle. Furthermore, in the tape 1f of (f), the entire first and second lateral edge surfaces 4c and 4d are each formed as a concave surface. Furthermore, in the tape 1g of (g), the first and second lateral edge surfaces 4c are each formed as convex surfaces between the first end 4a and the easily breakable portion 5a at the middle and between the second end 4b and the easily breakable portion 5a at the middle. Moreover, in the tape 1h of (h), the adhesive area A of the tape-shaped member 4 includes, in the end portions thereof on the first end 4a side and the second end 4b side, width uniform sections that are each formed with a width that is the same as a corresponding one of the adjacent holding areas H1 and H2. Furthermore, the width decreasing section and the width increasing section described above are formed in the portions interposed between the width uniform sections. Note that in the width decreasing section and the width increasing section, the first lateral edge surface 4c and the second lateral edge surface 4d form, in plan view, a straight inclined surface, and the width of the holding area H1 and the width of the holding area H2 are substantially the same.

In each of the above double eyelid forming tapes 1e to 1h, when extended in the longitudinal direction, the adhesive area A is sequentially stretched in two directions from the middle (the position where the tape width is the smallest) in the longitudinal direction towards the first end 4a and the second end 4b. Furthermore, a superior adhesiveness to the eyelid on the first end 4a side and the second end 4b side of the adhesive area A can be obtained, and when extended and stuck on the eyelid, adhesiveness to the eyelid at the end portions of the eye on the outer corner side 92 and the end portion of the eye on the inner corner side 93 can be both improved.

Note that the shape of the cosmetic eyelid-shaping tape according to the present invention is not necessarily limited to those described above, and it is only sufficient that the adhesive area A of the tape-shaped member 4 has only one section of the width decreasing section in which the width continuously decreases from the first end 4a side towards the second end 4b side, and does not have, on the above-described first end 4a side with respect to the width decreasing section, a portion in which the width is smaller than the largest width of the base material piece 2 in the width decreasing section (in other words, the largest width of the tape-shaped member 4 in the width decreasing section).

A second embodiment of the cosmetic eyelid-shaping tape according to the present invention will be described next with reference to Fig. 17. Note that the configurations and the functions and effects that are different from those of the first embodiment described above will be mainly described herein, and as for the configurations and the functions and effects that are practically the same, description will be omitted to avoid redundancy. Furthermore, in the second embodiment as well, description will be given by citing a piece of double eyelid forming tape as an example.

A piece of double eyelid forming tape 12 illustrated in Fig. 17 is, in place of the release sheet pieces 5 in the tape 1 described above, formed of films that has an easy release characteristics against the adhesive layers 3 described above, and is provided with release film pieces 9 that are stuck on the adhesive area A of the tape-shaped member 4 described above while covering the adhesive layers 3 described above and, and holding film pieces 4e that make each of the pair of holding areas H1 and H2 of the tape-shaped member 4 described above nonadhesive. Since the base material piece 2 constituting the tape-shaped member 4 is the same as the first embodiment described above, description will be omitted herein.

The release film pieces 9 described above are formed shorter than the tape-shaped member 4 described above and, for example, are formed of release paper laminated with a silicone resin. Furthermore, the holding film pieces 4e described above are stuck to the adhesive layers 3 of the tape-shaped member 4 described above in a fixed manner, form the pair of holding areas H1 and H2 described above, and, are formed of, for example, thin paper, resin film, or the like. Furthermore, the release film pieces 9 described above exposing a portion of each of the holding areas H1 and H2 described above on the first end 4a side and the second end 4b side described above extend in the longitudinal direction of the tape-shaped member 4. Note that the release film pieces 9 described above may have length that expose the entire holding areas H1 and H2 described above.

In using such a piece of double eyelid forming tape 12 according to the second embodiment, first, the exposed nonadhesive holding areas H1 and H2 of the tape-shaped member 4 described above are held with the fingertips, and the adhesive area A is pulled in the longitudinal direction. By so doing, simultaneously with the above-described adhesive area A of the tape-shaped member 4 being stretched, the release film pieces 9 and 9 described above are separated from both surfaces of the adhesive area A and, as a result, the adhesive area A is exposed and becomes visible. The subsequent method for use and the functions and effects are as described above; accordingly, in order to avoid redundant description, description thereof is omitted herein.

While the embodiments and modifications of the present invention have been described above, the claimed invention is not limited to the above and it goes without saying that various design changes can be made within the scope of the claimed invention.

For example, the cosmetic eyelid-shaping tape according to the present invention can be used as a piece of tape to form a bulge (a so-called periorbital puffiness) in the lower eyelid. In such a case, similar to the case described above in which the double eyelid is formed, the tape-shaped member 4 described above is pulled in the longitudinal direction and, in a state in which the adhesive area A thereof is kept stretched at the predetermined length, the adhesive area A is abutted against and stuck on the lower position of the lower eyelid where the bulge is to be formed. Subsequently, when the pull of the tape-shaped member 4 described above is released, the tape-shaped member 4 in the adhesive area A described above bites into the above position with the elastic contractility of the base material piece 2, and the constricted portion is formed in the above along the tape-shaped member 4. With the above, a bulge is formed on the upper portion of the constricted portion formed in the lower eyelid such that a three-dimensional effect is provided in the eye area. As described above, in a case in which the cosmetic eyelid-shaping tape according to the present invention is used as a piece of tape for forming a periorbital puffiness, the length of the tape-shaped member 4 may be made shorter than that of the double eyelid forming tape described above.

### Reference Signs List

- 1, 12: double eyelid forming tape (cosmetic eyelid-shaping tape)
- 2: base material piece
- 3: adhesive layer
- 4: tape-shaped member
- 4a: first end
- 4b: second end
- 4c: first lateral edge surface
- 4d: second lateral edge surface
- 4e: holding film piece
- 5: release sheet piece
- 5a: easily breakable portion
- 6: first base material layer
- 7: second base material layer
- 8: adhesion layer
- 90: constricted portion
- 91: fold
- 92: outer corner side of the eye
- 93: inner corner side of the eye
- A: adhesive area
- H1, H2: holding area

## Claims

1. A piece of cosmetic eyelid-shaping tape comprising:
a tape-shaped member including a base material piece capable of being stretched in a longitudinal direction, the base material being formed of a long and narrow synthetic resin that has elastic contractility when stretched, and an adhesive layer coated on both surfaces or one surface of the base material piece, the cosmetic eyelid-shaping tape being used for performing pseudo shaping on an eyelid by using the elastic contractility of the base material piece to form a constricted portion in the eyelid,
wherein the tape-shaped member includes, on a first end side and a second end side of the tape-shaped member in the longitudinal direction, a pair of holding areas to be held with fingertips, and an adhesive area between the pair of holding areas, the adhesive area to be stuck on the eyelid by being stretched to a predetermined length, and
wherein the adhesive area includes only a single width decreasing section in which a width of the base material piece continuously decreases from the first end side towards the second end side, and the adhesive area does not have, with respect to the width decreasing portion, a portion on the first end side that is smaller in width than a largest width of the base material piece in the width decreasing section.

2. The cosmetic eyelid-shaping tape according to Claim 1,
wherein an entire area of the adhesive area in the longitudinal direction is formed by the width decreasing section.

3. The cosmetic eyelid-shaping tape according to Claim 2,
wherein the tape-shaped member is formed in a triangle or a trapezoid in plan view in which a width becomes smaller from a first end towards a second end.

4. The cosmetic eyelid-shaping tape according to Claim 1,
wherein the base material piece includes at least a first base material layer, and
wherein the first base material layer of the base material piece is elongated when the adhesive area is stretched to the predetermined length, and has elastic contractility in an elongated state as well.

5. The cosmetic eyelid-shaping tape according to Claim 4,
wherein the base material piece is formed of a laminated body of the first base material layer formed of a polyolefin resin, and a second base material layer formed of an elastomer resin.

6. The cosmetic eyelid-shaping tape according to Claim 5,
wherein the first base material layer is formed of a low-density polyethylene resin, and the second base material layer is formed of a polyurethane resin.

7. The cosmetic eyelid-shaping tape according to Claim 1,
wherein the cosmetic eyelid-shaping tape is further formed of a synthetic resin that has easy release characteristics against the adhesive layer, and includes a release sheet piece that covers the adhesive layer and that is stuck on the holding areas and the adhesive area of the tape-shaped member, and
wherein the release sheet piece includes an easily breakable portion at a portion corresponding to the adhesive area, the easily breakable portion having a depth reaching a middle in a thickness direction while being provided so as to extend in the width direction, the easily breakable portion being broken by pulling in the longitudinal direction.

8. The cosmetic eyelid-shaping tape according to Claim 1,
wherein the cosmetic eyelid-shaping tape is further formed of a film having easy release characteristics against the adhesive layer, and includes a release film piece that covers the adhesive layer and is stuck on the adhesive area of the tape-shaped member,
wherein the pair of holding areas are nonadhesive, and
wherein the release film piece is formed shorter than the tape-shaped member, a portion or all of the holding areas being exposed on the first end side and the second end side, and the release film piece extends on the tape-shaped member.

9. A method for forming a double eyelid by using the cosmetic eyelid-shaping tape according to any one of Claims 1 to 8, the method comprising the following steps:
1. a step of stretching an adhesive area of the tape-shaped member to the predetermined length by holding and pulling a pair of holding areas of the tape-shaped member in the longitudinal direction;
2. a step of sticking a portion or all of the adhesive area stretched to the predetermined length in step 1 on an upper eyelid with the adhesive by abutment against the upper eyelid while the tape-shaped member is kept in a pulled state;
3. a step of forming a constricted portion along the adhesive area in the upper eyelid by releasing the pulling of the tape-shaped member and elastically shrinking a base material piece in the adhesive area that has been stuck on the upper eyelid; and
4. a step of forming a fold of the double eyelid by skin of the upper eyelid being bent back at the constricted portion when the upper eyelid is opened.

10. A method for producing the cosmetic eyelid-shaping tape according to any one of Claims 1 to 8, the method comprising the following steps:
1. a step of preparing an adhesive sheet on which an adhesive is coated on both surfaces or one surface of a base material sheet formed of a synthetic resin that forms the base material piece; and
2. a step of obtaining the cosmetic eyelid-shaping tape including the tape-shaped member by cutting the adhesive sheet with a cutting blade.

## Amended claims

### Amended claims under Art. 19.1 PCT

**1.** (Amended) A piece of cosmetic eyelid-shaping tape comprising:
a tape-shaped member including a base material piece capable of being stretched in a longitudinal direction, the base material being formed of a long and narrow synthetic resin that has elastic contractility when stretched, and an adhesive layer coated on both surfaces or one surface of the base material piece, the cosmetic eyelid-shaping tape being used for performing pseudo shaping on an eyelid by using the elastic contractility of the base material piece to form a constricted portion in the eyelid,
wherein the tape-shaped member includes, on a first end side and a second end side of the tape-shaped member in the longitudinal direction, a pair of holding areas to be held with fingertips, and an adhesive area between the pair of holding areas, the adhesive area to be stuck on the eyelid by being stretched to a predetermined length, and
wherein an entirety of the adhesive area in the longitudinal direction is formed by a width decreasing section in which a width of the base material piece continuously decreases from the first end side towards the second end side.

**12.** Cancelled)

**13.** The cosmetic eyelid-shaping tape according to Claim 2,
wherein the tape-shaped member is formed in a triangle or a trapezoid in plan view in which a width becomes smaller from a first end towards a second end.

**14.** The cosmetic eyelid-shaping tape according to Claim 1,
wherein the base material piece includes at least a first base material layer, and
wherein the first base material layer of the base material piece is elongated when the adhesive area is stretched to the predetermined length, and has elastic contractility in an elongated state as well.

**15.** The cosmetic eyelid-shaping tape according to Claim 4,
wherein the base material piece is formed of a laminated body of the first base material layer formed of a polyolefin resin, and a second base material layer formed of an elastomer resin.

**16.** The cosmetic eyelid-shaping tape according to Claim 5,
wherein the first base material layer is formed of a low-density polyethylene resin, and the second base material layer is formed of a polyurethane resin.

**17.** The cosmetic eyelid-shaping tape according to Claim 1,
wherein the cosmetic eyelid-shaping tape is further formed of a synthetic resin that has easy release characteristics against the adhesive layer, and includes a release sheet piece that covers the adhesive layer and that is stuck on the holding areas and the adhesive area of the tape-shaped member, and
wherein the release sheet piece includes an easily breakable portion at a portion corresponding to the adhesive area, the easily breakable portion having a depth reaching a middle in a thickness direction while being provided so as to extend in the width direction, the easily breakable portion being broken by pulling in the longitudinal direction.

**18.** The cosmetic eyelid-shaping tape according to Claim 1,
wherein the cosmetic eyelid-shaping tape is further formed of a film having easy release characteristics against the adhesive layer, and includes a release film piece that covers the adhesive layer and is stuck on the adhesive area of the tape-shaped member,
wherein the pair of holding areas are nonadhesive, and
wherein the release film piece is formed shorter than the tape-shaped member, a portion or all of the holding areas being exposed on the first end side and the second end side, and the release film piece extends on the tape-shaped member.

**19.** A method for forming a double eyelid by using the cosmetic eyelid-shaping tape according to any one of Claims 1 to 8, the method comprising the following steps:
1. a step of stretching an adhesive area of the tape-shaped member to the predetermined length by holding and pulling a pair of holding areas of the tape-shaped member in the longitudinal direction;
2. a step of sticking a portion or all of the adhesive area stretched to the predetermined length in step 1 on an upper eyelid with the adhesive by abutment against the upper eyelid while the tape-shaped member is kept in a pulled state;
3. a step of forming a constricted portion along the adhesive area in the upper eyelid by releasing the pulling of the tape-shaped member and elastically shrinking a base material piece in the adhesive area that has been stuck on the upper eyelid; and
4. a step of forming a fold of the double eyelid by skin of the upper eyelid being bent back at the constricted portion when the upper eyelid is opened.

**20.** A method for producing the cosmetic eyelid-shaping tape according to any one of Claims 1 to 8, the method comprising the following steps:
1. a step of preparing an adhesive sheet on which an adhesive is coated on both surfaces or one surface of a base material sheet formed of a synthetic resin that forms the base material piece; and
2. a step of obtaining the cosmetic eyelid-shaping tape including the tape-shaped member by cutting the adhesive sheet with a cutting blade.

Statement under Art. 19.1 PCT
Claim 1 of the Claims have clarified that, in the cosmetic eyelid-shaping tape, the entirety of the adhesive area in the longitudinal direction is formed with the width decreasing section in which the width of the base material piece continuously decreases from the first end side towards the second end side.

Conversely, the cited documents 1 to 3 do not disclose nor suggest whatsoever a configuration in which "an entirety of the adhesive area in the longitudinal direction is formed by a width decreasing section in which a width of the base material piece continuously decreases from the first end side towards the second end side". Moreover, the claimed invention employs the configuration described above to the "cosmetic eyelid-shaping tape for performing pseudo shaping on the eyelid by using the elastic contractility when the base material piece is stretched" and thus obtains the following noticeable function and effect that the above cited documents cannot derive. In other words, when the base material piece is stretched, the stretching gradually progresses from the second end side where the width is small towards the first end side where the width is large. In so doing, the adhesive layer coated on the base material piece becomes thinner in portions where the stretching has progressed more, and since the progress of the stretching is delayed more on the first end side where the width in the adhesive area is large, an adhesive layer that is thicker than that on the second end side remains on the first end side and, as a result, an outstanding adhesiveness to the skin can be obtained on the first end side where the width is larger.
